# EUROPÄISCHE PATENTANMELDUNG

(11) **EP 1 876 229 A1**
(43) Veröffentlichungstag der Anmeldung: **09.01.2008**
(21) Anmeldenummer: 07013112.3
(22) Anmeldetag: 04.07.2007
(51) Int. Cl.: C12M 1/107, B01F 7/00

(54) **Biogasanlage zur Fermentation von organischen Stoffen**

(30) Priorität: 05.07.2006 DE 202006010384 U
(71) Anmelder: U. T. S. Umwelt-Technik-Süd GmbH, 84419 Obertaufkirchen (DE)
(72) Erfinder: Bürger, Adam, 83527 Haag (DE)
(74) Vertreter: Neubauer, Hans-Jürgen

(57) **Zusammenfassung**

Die Erfindung betrifft eine Biogasanlage zur Fermentation von organischen Stoffen mit einem Fermenterbehälter, mit einem Serviceschacht und mit einer Rühreinheit, wobei der Serviceschacht als gasdichte Abdeckung domartig über einer in einer Fermenterbehälterabdeckung ausgebildeten Fermenteröffnung angebracht ist. Der Serviceschacht weist in wenigstens einer Schachtwand eine zu Servicezwecken öffenbare Schachtwandöffnung auf. Die Rühreinheit ist mittels einer Stelleinrichtung von einer Funktionsstellung im Fermenterbehälter durch die Fermenteröffnung nach oben in eine Servicestellung bewegbar und dort fixierbar, wobei die Rühreinheit einen Antriebsmotor mit einer etwa horizontal gerichteten Antriebswelle aufweist, auf der ein Rührflügelelement drehbewegungsübertragend angebracht ist, und zwar mit zwei an einem Nabenteil winkelversetzt angeordneten Rührflügeln. Erfindungsgemäß ist ein zweites Rührflügelelement (22) mit wenigstens einem Rührflügel (24, 24') lösbar, drehbewegungsübertagend und mit einem Winkelversatz des wenigstens einen Rührflügels (24, 24') zu den Rührflügeln (23, 23') des ersten Rührflügelelements (21) mit der Antriebswelle (19) verbunden.

## Beschreibung

Die Erfindung betrifft eine Biogasanlage zur Fermentation von organischen Stoffen mit einem Fermenterbehälter, einem Serviceschacht und einer Rühreinheit nach dem Oberbegriff des Anspruchs 1.

Eine bekannte, gattungsgemäße Biogasanlage zur Fermentation von organischen Stoffen (DE 197 14 342 C2) besteht unter anderem aus einem Fermenterbehälter mit einem Serviceschacht und mit einer Rühreinheit. Der Serviceschacht ist als gasdichte Abdeckung domartig über einer in einer Fermenterbehälterabdeckung ausgebildeten Fermenteröffnung angebracht und weist wenigstens an einer seitlichen Serviceschachtwand eine zu Servicezwecken öffenbare Schachtwandöffnung auf. Die Rühreinheit ist mittels einer Stelleinrichtung von einer Funktionsstellung im Fermenterbehälter durch die Fermenteröffnung nach oben in den Serviceschacht in eine Servicestellung bewegbar und dort fixierbar. Die Rühreinrichtung besteht aus einem Antriebsmotor mit einer etwa horizontal gerichteten Antriebswelle auf der ein Rührflügelelement drehbewegungsübertragend angebracht ist, wobei an einem Nabenteil in Drehrichtung winkelversetzt zwei Rührflügel angeordnet sind.

Konkret besteht hier die Stelleinrichtung aus einer vertikal im Fermenterbehälter angeordneten Stützstange an der über eine Führungseinrichtung die Rühreinheit höhenverstellbar gehalten ist. Die Stützstange, ragt mit ihrem oberen Stützstangenende in den Serviceschachtinnenraum, bzw. durch diesen weiter nach oben zur Serviceschachtaußenseite, so dass die Rühreinheit im ihre Servicestellung an der Stützstange nach oben in den Serviceschachtinnenraum verfahrbar ist. Im normalen Betrieb der Biogasanlage ist der Serviceschacht gasdicht über der zugeordneten Fermenteröffnung mit geschlossener Schachtwandöffnung angeordnet. Zur Durchführung von Reparatur- und/oder Wartungsarbeiten an der Rühreinheit kann diese gut und bequem zugänglich bei hoher Arbeitssicherheit und bei kontinuierlichem Weiterbetrieb der Biogasanlage in der Servicestellung im Serviceschacht durchgeführt werden. Insbesondere kann in der Servicestellung die Rühreinheit bei einer Erstmontage oder bei Reparatur- und/oder Wartungsarbeiten ein- oder ausgebaut werden.

Der Serviceschacht ist in einer konkreten Ausführung aus hochwertigem, korrosionsfestem Edelstahlblech gefertigt, da die Biogasatmosphäre andernfalls zu Korrosionsschäden am Serviceschacht führen würde. Die zu verwendenden Materialien sind somit relativ teuer. Weiter soll der Serviceschacht zwar die für Reparatur- und/oder Wartungsarbeiten erforderliche Größe für eine gute Zugänglichkeit zu der Rühreinheit aufweisen, jedoch aus Kostengründen bei der Herstellung, sowie für den Transport und die Montage möglichst klein gehalten werden.

Serviceschächte werden bei Fermenterbehältern mit Betondecken ebenso verwendet wie bei Fermenterbehältern mit an sich bekannten Zeltdächern. Bei solchen Zeltdachausführungen ist es erforderlich, den Serviceschacht auf einem Traggestell über einer Öffnung im Zeltdach anzubringen, wobei der Öffnungsrand des Zeltdachs dicht mit dem Serviceschacht zu verbinden ist. Ersichtlich ist gerade bei dieser Konstruktion ein relativ kleiner, gewichtsgünstiger Serviceschacht vorteilhaft.

Ein kleiner Serviceschacht mit einer relativ kleinen Schachtwandöffnung ist ersichtlich dann möglich, wenn die zugeordnete Rühreinheit und deren Rührflügel ebenfalls relativ klein sind. Eine bessere Durchmischung des Substrats im Fermenter mit höherer Rührleistung kann insbesondere bei hydraulisch betriebenen Rühreinheiten mit größeren und längeren Rührflügeln erreicht werden. Ein Rührflügelelement in der Art eines Rührflügelpropellers mit zwei gegenüberliegenden Rührflügeln kann bei einer üblichen Größe eines Serviceschachts bis zu einem Gesamtflügeldurchmesser von etwa 1,6 Metern durch die Schachtwandöffnung bewegt werden. Allerdings laufen solche großen, zweiflügeligen Rührflügelelemente sehr unruhig, insbesondere wenn sich darin Feststoffe verfangen und herumwickeln. Dreiflügelige Rührpropeller laufen dagegen ruhiger, können jedoch aus dem vorstehenden, selben Serviceschacht mit den gleichen Abmessungen der Schachtwandöffnung nur bis zu einem Gesamtflügeldurchmesser von ca. 1 Meter aus- und eingebaut werden. Mit einem solchen dreiflügeligen Rührpropeller wird zwar ein ruhigerer Lauf erreicht, jedoch wird wegen des reduzierten Gesamtflügeldurchmessers die Rührleistung gegenüber einem zweiflügeligen größeren Rührflügelpropeller reduziert.

Aufgabe der Erfindung ist es, eine gattungsgemäße Anordnung so weiterzubilden, dass bei gleicher Serviceschachtabmessung eine Rühreinheit mit ruhigem Lauf und hoher Rührleistung durch die Schachtwandöffnung eines relativ kleinen Serviceschachts einfach montierbar und demontierbar ist.

Diese Aufgabe wird mit den Merkmalen des Anspruchs 1 gelöst.

Gemäß Anspruch 1 wird auf ein erstes zweiflügeliges Rührflügelelement ein zweites Rührflügelelement mit wenigstens einem Rührflügel drehbewegungsübertragend und mit einem Winkelversatz des wenigstens einen Rührflügels zu den Rührflügeln des ersten Rührflügelelements aufgesetzt und lösbar fixiert.

Damit wird erreicht, dass die Rühreinheit mit wenigstens drei montierten Rührflügeln problemlos im Serviceschacht aufgenommen werden kann, wobei eine entsprechend große Fermenteröffnung in einer Betondecke oder einem Zeltdach unter dem Serviceschacht problemlos zur Verfügung gestellt werden kann. In dieser Position kann aber weder ein einstückiger dreiflügeliger noch ein einstückiger vierflügeliger Rührpropeller durch die relativ kleine Schachtwandöffnung aus dem Serviceschacht heraus oder in diesen hinein gebracht und auf der Antriebswelle montiert oder demontiert werden. Erfindungsgemäß wird dieses Problem dadurch gelöst, dass im Serviceschacht das zweite Rührflügelelement gelöst wird und als separates Teil problemlos durch die Schachtwandöffnung transportiert wird. Es verbleibt dann noch das zweite Rührflügelelement, mit dem auch bei relativ großen Gesamtflügeldurchmessern eine Montage oder Demontage problemlos möglich ist.

In einer konstruktiv einfachen Lösung ist der wenigstens eine Rührflügel des zweiten Rührflügelelements an einem Nabenring angebracht, der auf ein das erste Rührflügelelement überragendes Antriebswellenende verdrehfest aufsteckbar und fixierbar ist.

Bei einer besonders bevorzugten Ausführungsform nach Anspruch 3 mit hoher Laufruhe und hoher Rührleistung der Rühreinheit weist das erste Rührflügelelement zwei einander gegenüberliegende Rührflügel auf. Auch das zweite Rührflügelelement hat zwei einander gegenüberliegende Rührflügel. Die beiden Rührflügelelemente werden auf der Antriebswelle und/oder untereinander lösbar so montiert, dass alle Rührflügel in einer regelmäßigen vierflügeligen Anordnung um 90° winkelversetzt sind.

Alternativ dazu kann je nach den Gegebenheiten und Erfordernissen auch ein regelmäßiger dreiflügeliger, demontierbarer Rührflügelpropeller erreicht werden, wobei das erste Rührflügelelement zwei um etwa 120° winkelversetzte Rührflügel aufweist, und das zweite Rührflügelelement einen lösbaren Rührflügel hat, der so montierbar ist, dass alle drei Rührflügel um etwa 120° winkelversetzt sind.

Vorteilhaft wird der Winkelversatz der Rührflügel bei einer regelmäßigen vierflügeligen oder dreiflügeligen Anordnung durch die Geometrie der Bauteile vertauschungssicher bereits vorbestimmt. In einer einfachen Konstruktion nach Anspruch 5 weisen dabei das Nabenteil des ersten Rührflügelelements sowie der Nabenring des zweiten Rührflügelelements jeweils eine axial verlaufende Keil-Nut-Verbindung als Verdrehsicherung zur Antriebswelle auf. Die Eingriffelemente am Nabenteil und am Nabenring sind dabei so angeordnet, dass die vorbestimmte Winkellage der Rührflügel montagesicher vorgegeben ist.

Je nach den Erfordernissen und Gegebenheiten können nach Anspruch 6 die Rührflügel in einem bestimmten Umfang radial unterschiedlich lang und/oder unterschiedlich geformt sein. Insbesondere können in einer vierflügeligen Anordnung jeweils zwei gegenüberliegende Rührflügel die gleiche Form und Länge aufweisen.

Für eine einfache Montage und Demontage beider Rührflügelelemente wird mit Anspruch 7 vorgeschlagen, dass das Nabenelement des ersten Rührflügelelements und der Nabenring des zweiten Rührflügelelements auf der Antriebswelle unverdrehbar aufgesteckt sind und mittels einer endseitigen Spannschraubenmutter gegen einen Anschlagring der Antriebswelle gespannt sind.

Für eine axiale Fixierung und/oder eine Drehverbindung kann nach Anspruch 8 das Nabenelement des ersten Rührelements an einem festen Anschlagring der Antriebswelle angeschraubt sein. Der Nabenring des zweiten Rührflügelelements wird dann zweckmäßig am Nabenelement des ersten Rührflügelelements angeschraubt, wobei auch eine solche konstruktive Ausführungsform einfach montierbar und demontierbar ist.

Die Stelleinrichtung für die Höhenverstellung der Rühreinheit bis in den Serviceschacht ist in an sich bekannter Weise nach Anspruch 1 als Stützstange ausgebildet, die mit ihrem oberen Ende in den Serviceschachtinnenraum, ggf. durch diesen nach oben außen ragt, wobei an der Stützstange die Rühreinheit höhenverstellbar geführt ist.

Alternativ kann nach Anspruch 10 die Stelleinrichtung auch als Schwenkarm ausgeführt werden, der beispielsweise an einer Fermenterinnenwand angelenkt ist.

Anhand einer Zeichnung wird die Erfindung mit weiteren Einzelheiten erläutert.

Es zeigen:
- Fig. 1: einen schematischen Schnitt durch einen Fermenterbehälter einer Biogasanlage mit einem Serviceschacht
- Fig. 2: eine perspektivische Darstellung eines Serviceschachts, und
- Fig. 3: eine schematische Explosionsdarstellung eines Rührpropellers.

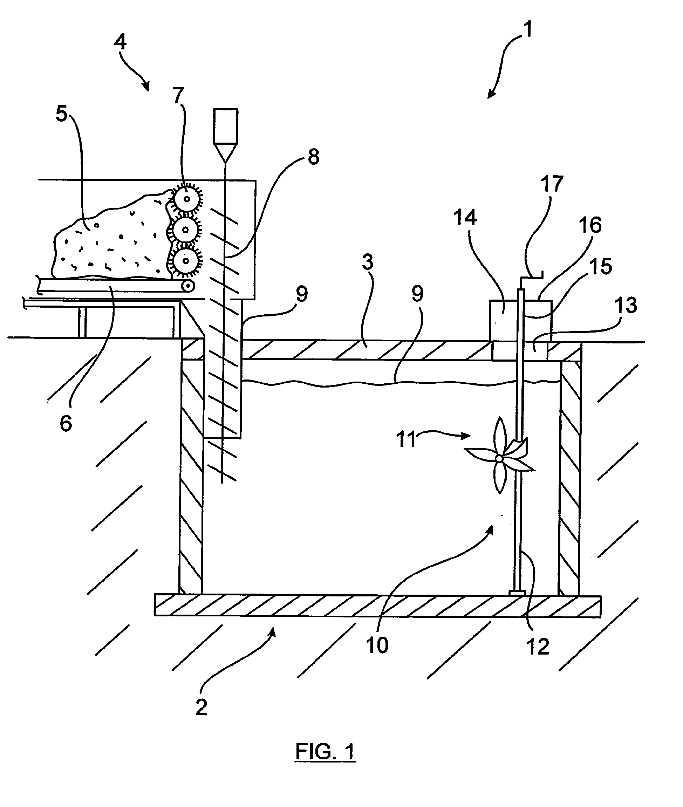

In Fig. 1 ist ein Schnitt durch einen Teil einer Biogasanlage 1 zur Fermentation von organischen Stoffen dargestellt mit einem Fermenterbehälter aus Betonwänden, wobei auch die Fermenterbehälterabdeckung als Betondecke 3 ausgebildet ist. Zudem ist schematisch eine Feststoffbeschickungsanlage 4 gezeigt, bei der Biofeststoffe 5 über ein Förderband 6 gegen Zerkleinerungswalzen 7 geführt werden und anschließend über eine vertikale Beschickungsschnecke 8 in einem Tauchrohr 9 in den Fermenterbehälter 2 unter den Flüssigkeitsspiegel 9 eingebracht werden.

Zudem ist eine Rühreinrichtung 10 gezeigt, bei der eine Rühreinheit 11 an einer Stützstange 12 höhenverstellbar mittels einer (nicht dargestellten) Stelleinrichtung geführt und gehalten ist. Im Bereich der Stützenstange 12 ist in der Betondecke 3 eine Fermenteröffnung 13 ausgebildet, über der ein Serviceschacht 14 dicht angebracht ist. Der Serviceschacht 14 hat die Form eines Quaders mit Seitenwänden und einer Deckenwand 16 und ist unten über der Fermenteröffnung 13 offen. Die Stützstange 12 ist mit ihrem oberen Stützstangenende 15 in den Serviceschachtinnenraum geführt und ragt gasdicht und verdrehbar durch die Deckenwand 16 des Serviceschachts 14, wo zudem eine Handkurbel 17 für eine Drehverstellung der Stützstange 12 und der daran geführten Rühreinheit 11 angebracht ist.

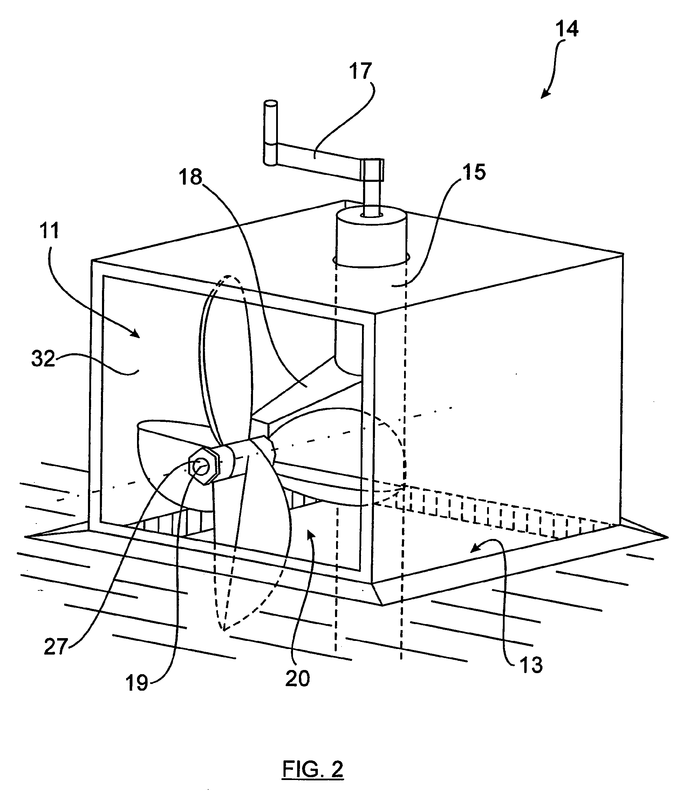

In Fig. 2 ist der Serviceschacht 14 vergrößert und perspektivisch gezeigt, ebenso das obere Stützstangenende 15 mit der Handkurbel 17. Die Rühreinheit 11 ist hier in ihre Servicestellung nach oben in den Serviceschacht 14 eingefahren.

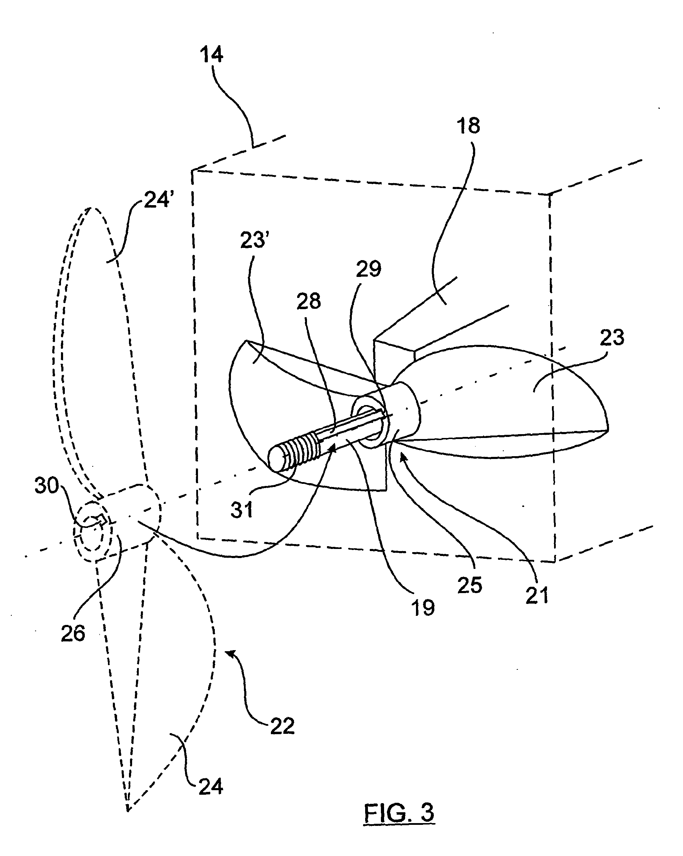

Die Rühreinheit 11 besteht aus dem Antriebsmotor 18, hier einem Hydraulikmotor mit einer horizontal gerichteten Antriebswelle 19. Weiter umfasst die Rühreinheit 11 einen Rührpropeller 20, der wie in Fig. 3 näher gezeigt ist aus einem hinteren Rührelement 21 und einem vorderen Rührelement 22 lösbar zusammengesetzt ist. Beide Rührelemente 21, 22 sind zweiflügelig mit gegenüberliegenden Rührflügeln 23, 23' sowie 24, 24' ausgeführt. Die Rührflügel 23, 23' des ersten auf der Antriebswelle 19 hinteren Rührelements 21 sind an einem Nabenteil 25 fest angebracht. Ebenso sind die Rührflügel 24, 24' des zweiten, auf der Antriebswelle 19 vorderen Rührflügelelements 22 an einem Nabenring 26 fest angebracht.

Wie aus Fig. 3 ersichtlich können beide Rührflügelelemente 21, 22 von der Antriebswelle 19 einfach demontiert werden und sind dazu dort mit dem Nabenteil 25 und mit dem Nabenring 26 aufgesteckt und mit einer endseitigen Spannmutter 27 axial gesichert. Als Mitnehmer und Verdrehsicherung ist an der Antriebswelle 19 eine axiale Keil-Nut-Verbindungen vorgesehen, bei der ein Längskeil 28 auf der Antriebswelle 19 angebracht ist. Zugeordnete Nuten 29, 30 sind an den Innenseiten des Nabenteils 25 bzw. des Nabenrings 26 eingelassen. Die Nuten 29 und 30 sind bezüglich der zugeordneten Radialausrichtung der Rührflügel 23, 23' bzw. 24, 24' um 90° versetzt, so dass nach der Montage die vier Rührflügel 23, 23', 24, 24' in Umfangsrichtung jeweils um 90°-winkelversetzt sind und eine regelmäßige vierflügelige Anordnung bilden. Die Antriebswelle 19 trägt am freien Ende ein Gewinde 31 auf das die Spannmutter 27 aufgeschraubt ist.

In Fig. 2 ist das Wandelement der nach vorne weisenden Seitenwand des Serviceschachts 14 abgenommen, so dass die Schachtwandöffnung 32 für Service- und Reparaturarbeiten an der Rühreinheit 11 freigegeben ist. Insbesondere soll in der gezeigten Servicestellung der Rühreinheit 11 ein einfacher Austausch des Rührpropellers 20 möglich sein.

Die Rühreinheit 11 kann durch die Fermenteröffnung 13 problemlos nach oben in den Serviceschacht 14 hineingezogen werden. Bei der vorgegebenen Größe des Serviceschachts 14 und der Schachtwandöffnung 32 könnte der vierflügelige Rührpropeller 20 in einer einstückigen Ausführung mit einer Nabe und daran fest angebrachten vier Rührflügeln nicht aus der Schachtwandöffnung 32 herausbefördert werden, da diese dafür zu klein ist. Durch die zweiteilige Ausführung des Rührpropellers 20 kann aber im Serviceschacht 14 das vordere Rührelement 22 gelöst und von der Antriebswelle 19 abgezogen werden. Damit ist eine Drehung und diagonale Raumstellung des Rührelements 22 möglich, dieses problemlos aus der ansonsten zu kleinen Schachtwandöffnung 32 herauszuziehen. Ebenso kann dann das andere Rührelement 21 von der Antriebswelle 19 abgezogen werden und durch geeignetes Verkippen aus der Schachtwandöffnung 32 herausbewegt werden. Bei einer Montage der Rühreinheit im Serviceschacht 14 wird in umgekehrter Reihenfolge verfahren.

## Patentansprüche

1. Biogasanlage zur Fermentation von organischen Stoffen
mit einem Fermenterbehälter, mit einem Serviceschacht und mit einer Rühreinheit, wobei
der Serviceschacht als gasdichte Abdeckung domartig über einer in einer Fermenterbehälterabdeckung ausgebildeten Fermenteröffnung angebracht ist, und der Serviceschacht in wenigstens einer Schachtwand eine zu Servicezwecken öffenbare Schachtwandöffnung aufweist,
die Rühreinheit mittels einer Stelleinrichtung von einer Funktionsstellung im Fermenterbehälter durch die Fermenteröffnung nach oben in eine Servicestellung bewegbar und dort fixierbar ist, und
die Rühreinheit einen Antriebsmotor mit einer etwa horizontal gerichteten Antriebswelle aufweist, auf der ein Rührflügelelement drehbewegungsübertragend angebracht ist mit zwei an einem Nabenteil winkelversetzt angeordneten Rührflügeln,
**dadurch gekennzeichnet,**
**dass** ein zweites Rührflügelelement (22) mit wenigstens einem Rührflügel (24, 24') lösbar, drehbewegungsübertagend und mit einem Winkelversatz des wenigstens einen Rührflügels (24, 24') zu den Rührflügeln (23, 23') des ersten Rührflügelelements (21) mit der Antriebswelle (19) verbunden ist.

2. Biogasanlage nach Anspruch 1, **dadurch gekennzeichnet, dass** der wenigstens eine Rührflügel (24, 24') des zweiten Rührflügelelements (22) an einem Nabenring (26) angebracht ist, der auf ein das Nabenteil (25) des ersten Rührflügelelements überragendes Antriebswellenende verdrehfest aufsteckbar und fixierbar ist.

3. Biogasanlage nach Anspruch 1 oder 2, **dadurch gekennzeichnet, dass** das erste Rührflügelelement (21) zwei aneinander gegenüberliegende Rührflügel (23, 23') aufweist, und
dass das zweite Rührflügelelement (22) ebenfalls zwei einander gegenüberliegende Rührflügel (24, 24') aufweist, wobei alle vier Rührflügel (23, 23', 24, 24') in der montierten Stellung gegeneinander winkelversetzt, vorzugsweise jeweils gegeneinander um 90° winkelversetzt in einer vierflügeligen Anordnung montiert sind.

4. Biogasanlage nach Anspruch 1 oder 2, **dadurch gekennzeichnet, dass** das erste Rührflügelelement (21) zwei um etwa 120° versetzte Rührflügel aufweist und das zweite Rührflügelelement (22) einen Rührflügel aufweist, wobei in der montierten Stellung alle Rührflügel in einer dreiflügeligen Anordnung jeweils um etwa 120° winkelversetzt montiert sind.

5. Biogasanlage nach einem der Ansprüche 1 bis 4, **dadurch gekennzeichnet, dass** die Antriebswelle (19) und das Nabenteil (25) des ersten Rührflügelelements (21) sowie der Nabenring (26) des zweiten Rührflügelelements (22) eine axiale Keil-Nut-Verbindung (28, 29, 30) als Verdrehsicherung zur Antriebswelle (19) aufweisen und die Eingriffelemente (29, 30) am Nabenteil (25) und Nabenring (26) so angeordnet sind, dass die gewünschte Winkellage der Rührflügel (23, 23', 24, 24') zueinander vorbestimmt ist.

6. Biogasanlage nach einem der Ansprüche 1 bis 5, **dadurch gekennzeichnet, dass** die Rührflügel radial unterschiedlich lang und/oder unterschiedlich geformt sind, wobei vorzugsweise in einer vierflügeligen Anordnung jeweils zwei gegenüberliegende Rührflügel die gleiche Form und Länge aufweisen.

7. Biogasanlage nach einem der Ansprüche 1 bis 6, **dadurch gekennzeichnet, dass** das Nabenelement (25) und der Nabenring (26) auf der Antriebswelle (19) aufgesteckt sind und mittels einer endseitigen Spannschraubenmutter (27) gegen einen Anschlagring der Antriebswelle (19) gespannt sind.

8. Biogasanlage nach einem der Ansprüche 1 bis 6, **dadurch gekennzeichnet, dass** das Nabenelement (25) an einem Anschlagring der Antriebswelle (19) angeschraubt ist und der Nabenring (26) am Nabenelement (25) angeschraubt ist.

9. Biogasanlage nach einem der Ansprüche 1 bis 8, **dadurch gekennzeichnet, dass** die Stelleinrichtung eine im Fermenterbehälter (2) vertikal gehaltene und mit einem oberen Stützstangenende (15) in den Serviceschacht-Innenraum ragende Stützstange (12) ist, an der die Rühreinheit (11) höhenverstellbar geführt ist.

10. Biogasanlage nach einem der Ansprüche 1 bis 8, **dadurch gekennzeichnet, dass** die Stelleinrichtung ein Schwenkarm ist.
